(19) Europäisches Patentamt / European Patent Office / Office européen des brevets

(11) **EP 4 291 084 B1**

(12) **EUROPEAN PATENT SPECIFICATION**

(45) Date of publication and mention
of the grant of the patent:
**11.03.2026 Bulletin 2026/11**

(21) Application number: **21933520.5**

(22) Date of filing: **27.03.2021**

(51) International Patent Classification (IPC):
*A61B 5/0205* (2006.01)    *A61B 5/0535* (2021.01)
*A61B 5/11* (2006.01)    *A61B 5/26* (2021.01)
*A61B 5/00* (2006.01)

(52) Cooperative Patent Classification (CPC):
**A61B 5/0205; A61B 5/02416; A61B 5/0535;
A61B 5/0537; A61B 5/1102; A61B 5/26;
A61B 5/304; A61B 5/332; A61B 5/6825;
A61B 5/6829; A61B 5/6887; G01G 19/50;**
A61B 5/02125; A61B 5/1036; A61B 5/6843;

(Cont.)

(86) International application number:
**PCT/CN2021/083455**

(87) International publication number:
**WO 2022/204833 (06.10.2022 Gazette 2022/40)**

(54) **WEIGHING SCALE FOR MONITORING BIOMETRIC DATA OF USER**

WAAGE ZUR ÜBERWACHUNG BIOMETRISCHER DATEN EINES BENUTZERS

BALANCE PERMETTANT DE SURVEILLER DES DONNÉES BIOMÉTRIQUES D'UN UTILISATEUR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**20.12.2023 Bulletin 2023/51**

(73) Proprietor: **Huawei Technologies Co., Ltd.
Shenzhen, Guangdong 518129 (CN)**

(72) Inventors:
• **VISSAC, Virginie
80992 Munich (DE)**
• **ZHU, Yu
80992 Munich (DE)**
• **REN, Huichao
Shenzhen, Guangdong 519128 (CN)**

(74) Representative: **Gill Jennings & Every LLP
The Broadgate Tower
20 Primrose Street
London EC2A 2ES (GB)**

(56) References cited:
WO-A1-2019/134032    CN-A- 106 333 660
CN-A- 109 820 504    CN-U- 205 597 918
US-A1- 2012 089 000    US-A1- 2013 289 889
US-A1- 2016 038 037    US-A1- 2016 095 522

(52) Cooperative Patent Classification (CPC): (Cont.)
A61B 2562/0247; A61B 2562/0252

**Description**

<u>TECHNICAL FIELD</u>

The present disclosure relates to a weighing scale for monitoring biometric data of a user. More specifically, the invention relates to a weighing scale monitoring biometric data of a user for providing a cardiovascular health assessment of the user.

<u>BACKGROUND</u>

Cardiovascular diseases are still one of the major causes of death worldwide, accounting for 31% of total death happening in the world according to the World Health Organization (WHO) in 2016. The majority of these diseases are caused by a poor diet and therefore could be prevented. However, because the majority of the examinations targeting the heart are done in medical facilities, the patients who are detected with a cardiovascular disease usually already experienced the first symptoms meaning that the body already suffered the first damages. If those diseases were to be detected earlier, the patient could change its way of life to improve his/her cardiovascular health before this may happen.

For instance, Peripheral Arterial Diseases (PAD) concern 200 million people in the world and about 20% of the population aged of 80 years old or older. This disease is a circulatory problem in which narrowed arteries reduce the blood flow to the limbs. This disease generally concerns the legs but can also concern the arms. 90% of the cases of PAD are caused by Atherosclerosis and in 50% of the cases this disease is asymptomatic. The conventional method to diagnose PAD is to use the Brachial-Ankle pressure index calculated by measuring the blood pressure in the four limbs and calculating a ratio between arms and legs that indicates if PAD is present. In a second stage Doppler and Ultrasound imaging can be used to confirm the diagnostic. Therefore, it is easily understood that these examinations will not be done without the presence of a specific risk of developing the disease or the presence of symptoms.

US 2016/095522 A1 discloses a user-platform apparatus, such as a bodyweight sensing scale, which includes a heart/cardiogram sensor which is used to detect heart and vascular characteristics of a user, and provide a cardiogram output indicative of the detected cardiovascular characteristics. The cardiogram output can be used for various purposes, such as detecting arterial stiffness and/or aging.

US 2016/038037 A1 discloses an arrangement of devices configured and arranged to monitor physiological parameters while the user is standing on a platform region of the device, and communicate an assessed fitness to the user as feedback. Further specific embodiments concern methods of monitoring physiological parameters of a user using the apparatus, assessing the fitness of the user based on one or more of the physiological parameters, and communicating the assessed fitness to the user as feedback.

US 2013/289889 A1 discloses a system comprising a biometric monitoring device including a housing including a platform to receive at least one foot of the user, a body weight sensor to generate body weight data, processing circuitry to calculate user weight data which corresponds to the user's weight, using the body weight data, and communication circuitry to: (a) receive user identification data which identifies the user or a portable activity monitoring device, and (b) transmit the user weight data to data storage associated with the user identification data.

WO 2019/134032 A1 discloses a multi-functional health monitoring mat which enables the measurement of- but not limited to the following parameters: Weight, Plantar pressure, Bio-impedance for body composition, and Cardiovascular based measurements, such as: Electrocardiogram (ECG), Ballistocardiogram (BCG) from which additional health indicating analytics can be further extracted.

<u>SUMMARY</u>

It is an objective of the present disclosure to provide a consumer device allowing for early detection of cardiovascular diseases.

The foregoing and other objectives are achieved by the subject matter of the independent claim. Further implementation forms are apparent from the dependent claims, the description and the figures. The invention is defined by the appended claims.

A weighing scale for monitoring biometric data of a user is provided. The weighing scale comprises a first group, i.e. first subset of a plurality of electrodes configured to be brought into contact with respective portions of a first hand of the user and a second group, i.e. a second subset of the plurality of electrodes configured to be brought into contact with respective portions of a second hand of the user. The weighing scale further comprises one or more handles configured to be gripped by the first and second hand of the user, wherein the first group of the plurality of electrodes and the second group of the plurality of electrodes are arranged on the one or more handles. In an embodiment the one or more handles may be provided by one or more handle bars. The weighing scale further comprises a base, i.e. platform configured to support the user standing on the base and determine a weight of the user. In an embodiment, the base may comprise a plate defining a top surface for supporting the user standing thereon.

A first portion of a top surface of the base comprises a third group of the plurality of electrodes configured to be brought into contact with a first bare foot of the user and a second portion of the top surface of the base comprises a fourth group of the plurality of electrodes configured to be brought into contact with a second bare foot of the user.

The weighing scale further comprises a processing circuitry connected to the plurality of electrodes and configured to obtain a plurality of biometric signals of the user, including one or more electrocardiogram, ECG, signals for one or more ECG leads, in particular 6 ECG leads, defined by, i.e. between the plurality of electrodes, a plurality of photoplethysmography (PPG) and/or impedance plethysmography (IPG) signals for the hands and the feet of the user for determining local blood volume variations in each limb of the user.

The processing circuitry of the weighing scale is further configured to determine, based on the plurality of biometric signals, a respective pulse arrival time, PAT, and to determine, based on the PAT, an approximated pulse wave velocity, PWV, for the first and second hand and the first and second foot of the user for estimating a peripheral arterial disease, PAD, risk of the user.

Thus, an everyday consumer device in the form of a weighing scale is provided allowing for early detection of cardiovascular diseases by estimating the PAD risk of the user. The weighing scale may embed additional measurements that can complete the cardiovascular health assessment of the user, such as for opportunistic detection of arythmias as well as heart morphology analysis by cardiac axis determination.

In a further possible implementation form, the plurality of biometric signals of the user further include one or more ballistocardiography, BCG, signals of the user, wherein the processing circuitry is further configured to obtain the one or more BCG signals on the basis of rapid weight variations of the user determined by the base.

In a further possible implementation form, the processing circuitry of the weighing scale is further configured to determine a pre-ejection period on the basis of the one or more ECG signals and the one or more BCG signals.

In a further possible implementation form, the processing circuitry of the weighing scale is further configured to determine a pulse transit time, PTT, and the PWV for the first and second hand and the first and second foot of the user for estimating the peripheral arterial disease, PAD, risk of the user.

In a further possible implementation form, the base further comprises at least one light sensor configured to detect the position of the feet of the user relative to the third group and the fourth group of the plurality of electrodes.

In a further possible implementation form, the base of the weighing scale comprises one or more load cells, in particular 4 load cells configured to determine the weight of the user.

In a further possible implementation form, the weighing scale further comprises a user interface and/or a communication interface configured to output information associated with one or more of the plurality of biometric signals to the user and/or configured to receive information about a height, age and/or sex of the user. The information about the height, age and/or sex of the user may be used by the processing circuitry for estimating the peripheral arterial disease, PAD, risk of the user.

In a further possible implementation form, the third group of the plurality of electrodes comprises at least 4 electrodes and/or the fourth group of the plurality of electrodes comprises at least 4 electrodes for local IPG measurement on each foot of the user.

The first group of the plurality of electrodes comprises at least 5 electrodes and/or the second group of the plurality of electrodes comprises at least 5 electrodes, for simultaneous local IPG measurement on each hand and ECG between the hands of the user.

In a further possible implementation form, the one or more handles comprises at least one PPG sensor for each handle and wherein the first group of the plurality of electrodes comprises at least 1 electrode and/or wherein the second group of the plurality of electrodes comprises at least 1 electrode for simultaneous measurement of ECG between the hands and PPG on each hand.

In a further possible implementation form, the processing circuitry is further configured to determine a first position of the first hand of the user and a second position of the second hand of the user on the one or more handles and to select, based on the first position and the second position, one or more of the at least 5 electrodes of the first group of the plurality of electrodes and/or one or more of the at least 5 electrodes of the second group of the plurality of electrodes for obtaining the plurality of biometric signals of the user.

In a further possible implementation form, the base of the weighing scale further comprises a pressure sensor array configured to detect the position of the first feet and the second feet of the user on the base.

In a further possible implementation form, the processing circuitry of the weighing scale is further configured to determine, based on the plurality of biometric signals, i.e. the plurality of ECG signals and the plurality of IPG and/or PPG signals, the pulse wave velocity, PWV, in the trunk of the user.

In a further possible implementation form, the processing circuitry of the weighing scale is configured to obtain the plurality of ECG signals for at least 6 ECG leads defined by, i.e. between the plurality of electrodes and to determine, based on the plurality of ECG signals, a heart morphology of the user and/or enable an opportunistic detection of arrythmias.

Details of one or more embodiments are set forth in the accompanying drawings and the description below. Other features, objects, and advantages will be apparent from the description, drawings, and claims.

BRIEF DESCRIPTION OF THE DRAWINGS

In the following, embodiments of the present disclosure are described in more detail with reference to the attached figures and drawings, in which:

Fig. 1 is a perspective view of a weighing scale according to an embodiment for determining a plurality of biometric signals of a user;

Fig. 2 is a more detailed top view of a weighing scale according to an embodiment;

Fig. 3 is a more detailed perspective view of the handles of a weighing scale according to an embodiment;

Fig. 4 is a more detailed view of the handles of a weighing scale according to a further embodiment;

Fig. 5 is a sequence diagram illustrating a sequence of biometric signals acquired by a weighing scale according to an embodiment;

Fig. 6 is a diagram illustrating exemplary biometric signals acquired by a weighing scale according to an embodiment, including a ECG, BCG and IPG signal;

Fig. 7 is a diagram illustrating in more detail exemplary biometric signals acquired by a weighing scale according to an embodiment;

Fig. 8a is a more detailed perspective view of the handles of a weighing scale according to a further embodiment;

Fig. 8b shows one of the weighing scale of figure 8a in contact with the hand of a user;

Fig. 9 is a diagram illustrating schematically a circuitry implemented by a weighing scale according to an embodiment for acquiring biometric signals;

Fig. 10 is a perspective view of the handles of a weighing scale according to further embodiments;

Fig. 11 is a diagram illustrating schematically a circuitry implemented by a weighing scale according to an embodiment for acquiring a biometric signal;

Fig. 12 is a diagram illustrating schematically a base of a weighing scale according to an embodiment;

Fig. 13 is a diagram illustrating schematically a base of a weighing scale according to an embodiment including a processing circuitry for processing a plurality of biometric signals;

Fig. 14 is a diagram illustrating schematically a base of a weighing scale according to a further embodiment;

Fig. 15 is a diagram illustrating schematically a base of a weighing scale according to a further embodiment.

In the following identical reference signs refer to identical or at least functionally equivalent features.

DETAILED DESCRIPTION OF THE EMBODIMENTS

In the following description, reference is made to the accompanying figures, which form part of the disclosure, and which show, by way of illustration, specific aspects of embodiments of the present disclosure or specific aspects in which embodiments of the present disclosure may be used. It is understood that embodiments of the present disclosure may be used in other aspects and comprise structural or logical changes not depicted in the figures. The following detailed description, therefore, is not to be taken in a limiting sense, and the scope of the present invention is defined by the appended claims.

For instance, it is to be understood that a disclosure in connection with a described method may also hold true for a corresponding device or system configured to perform the method and vice versa. For example, if one or a plurality of specific method steps are described, a corresponding device may include one or a plurality of units, e.g. functional units, to perform the described one or plurality of method steps (e.g. one unit performing the one or plurality of steps, or a plurality of units each performing one or more of the plurality of steps), even if such one or more units are not explicitly described or illustrated in the figures. On the other hand, for example, if a specific apparatus is described based on one or a plurality of units, e.g. functional units, a corresponding method may include one step to perform the functionality of the one or plurality of units (e.g. one step performing the functionality of the one or plurality of units, or a plurality of steps each performing the functionality of one or more of the plurality of units), even if such one or plurality of steps are not explicitly described or illustrated in the figures. Further, it is understood that the features of the various exemplary embodiments and/or aspects described herein may be combined with each other, unless specifically noted otherwise.

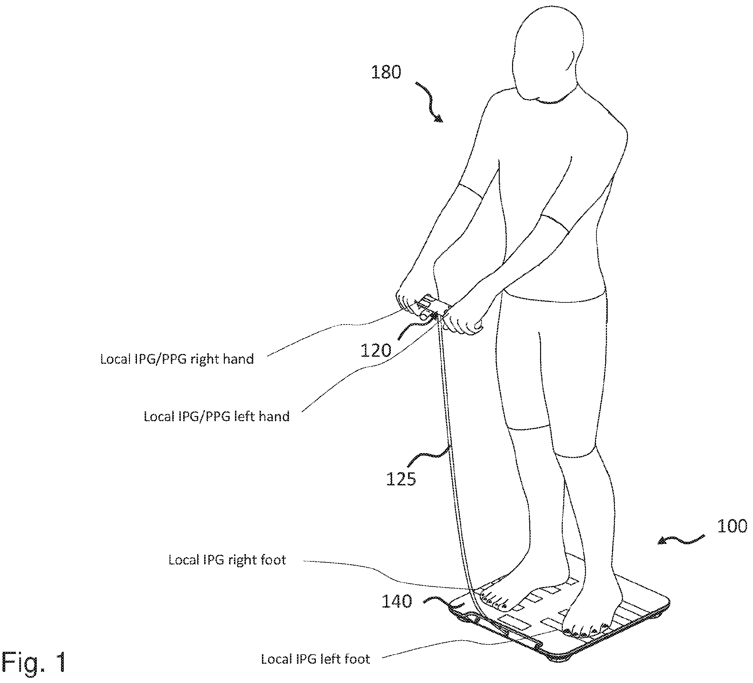

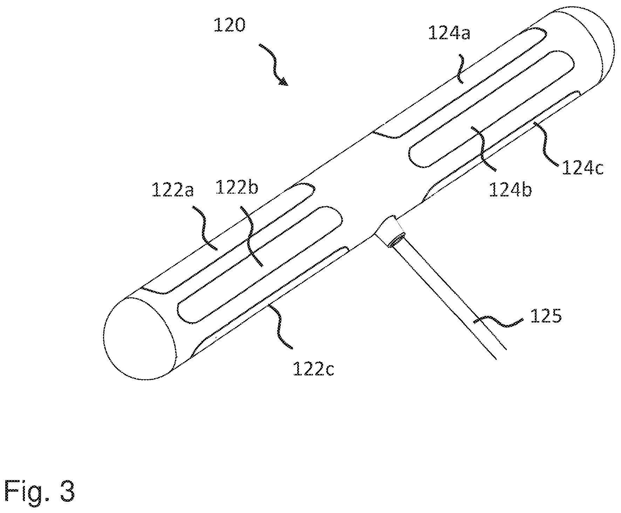

Figure 1 is a schematic diagram of a weighing scale 100 according to an embodiment for determining a plurality of biometric signals of a user 180. The weighing scale 100 comprises one or more handles 120, such as one or more handle bars 120 (as illustrated in figure 1), configured to be gripped by the first and second hand of the user 180. As will be described in more detail below and as shown, for instance, in figures 3, 4, 8 and 11, the weighing scale 100 comprises a first group 122a-g of a plurality of electrodes configured to be brought into contact with the first hand of the user 180 and a second group 124a-dg of the plurality of electrodes configured to be brought into contact with a second hand of the user 180. To this end, the first group 122a-g of the plurality of electrodes and the second group 124a-g of the plurality of electrodes are arranged on the one or more handles 120, e.g. the one or more handle bars 120.

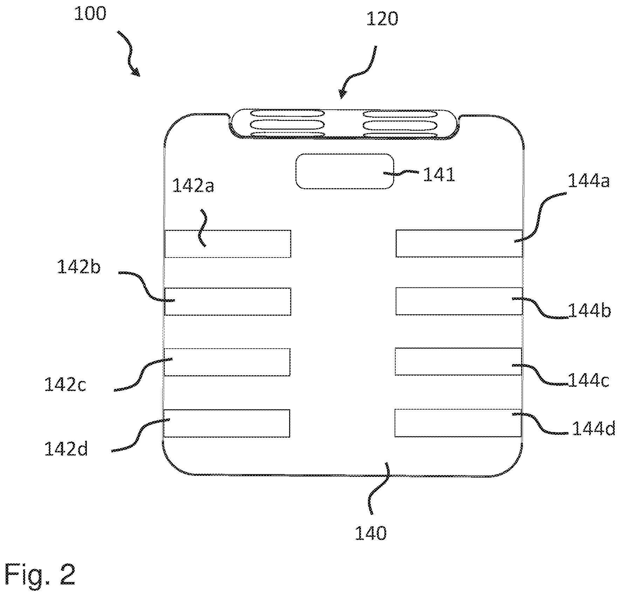

As illustrated in figure 1 and in more detail in figure 2, the weighing scale 100 further comprises a base 140 (also referred to as weighing platform 140) configured to support the user 180 standing thereon and to determine a weight of the user 180. A first portion of a top surface of the base 140 comprises a third group 142a-f of the plurality of electrodes configured to be brought into contact with a first bare foot of the user 180 standing on the base 140 and a second portion of the top surface of the base 140 comprises a fourth group 144a-f of the plurality of electrodes configured to be brought into contact with a second foot of the user 180. The weighing scale 100 may further comprises a user interface, such as a display 141 arranged on the top surface of the base 140 (as shown in figure 2) configured to output information associated with one or more of the plurality of biometric signals to the user 180 and/or a communication interface, such as a wireless interface, configured to receive information about a height, age and/or sex of the user 180. As illustrated in figure 1, the base 140 may be connected to the one or more handles 120 by a wiring 125.

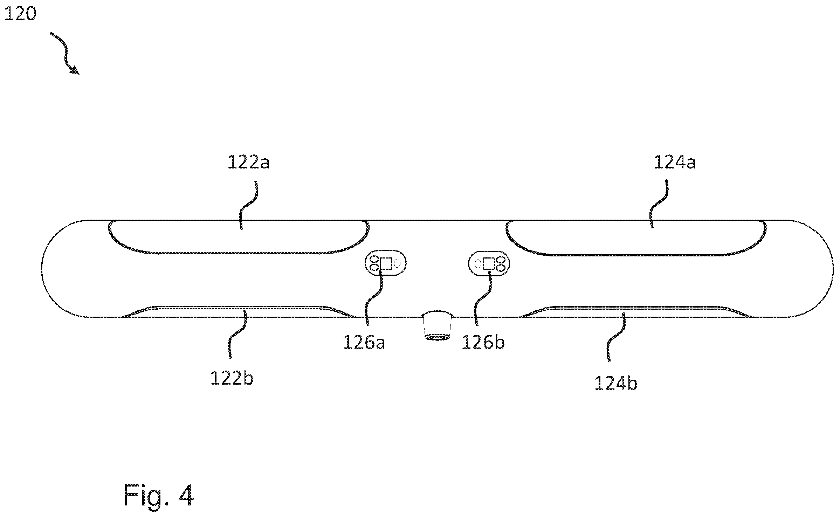

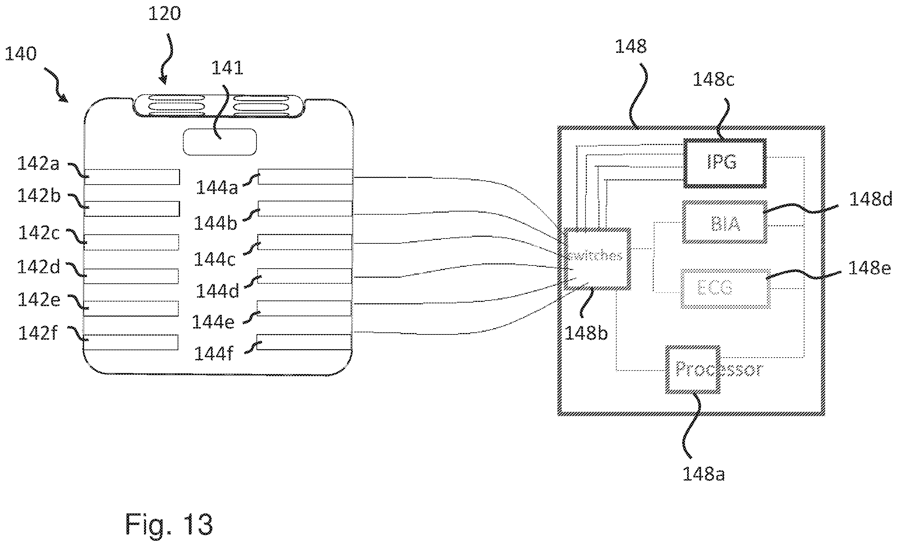

The weighing scale 100 further comprises a processing circuitry 148a (an embodiment thereof is shown in figure 13) configured to obtain a plurality of biometric signals of the user 180, including one or more electrocardiogram, ECG, signals for one or more ECG leads defined by the plurality of electrodes and a plurality of photoplethysmography (PPG) and/or impedance plethysmography (IPG) signals for the hands and the feet of the user 180 for determining local blood volume variations. In an embodiment, the first group 122a-g of the plurality of electrodes comprises at least 5 electrodes 122a-g and/or the second group 124a-g of the plurality of electrodes comprises at least 5 electrodes 124a-g for simultaneous measurement of local IPG on each hand and ECG between the hands of the user 180. In an embodiment, as illustrated in figure 4, the one or more handles 120 comprise at least one PPG sensor, such as LED diode and a light sensor 126a,b, for each handle 120, wherein the first group 122a-g of the plurality of electrodes comprises at least 1 electrode 122a-g and/or wherein the second group 124a-g of the plurality of electrodes comprises at least 1 electrode 124a-g for simultaneous measurement of ECG between the hands and PPG on each hand of the user 180.

As will be described in more detail below, the processing circuitry 148a of the weighing scale 100 is further configured to determine, based on the plurality of biometric signals, a pulse arrival time, PAT, and to determine, based on the PAT, a pulse wave velocity, PWV, for the first and second hand and the first and second foot of the user 180 for estimating a peripheral arterial disease, PAD, risk of the user 180. As will be appreciated, the pulse wave velocity, PWV, is the speed of the pulse pressure wave displacement. Looking at the speed at which a pressure wave travels in the artery enables to learn information on these arteries such as stiffening, blocking or even blood pressure. However, the PWV is not the same throughout the body, i.e. it changes from trunk to arms and legs.

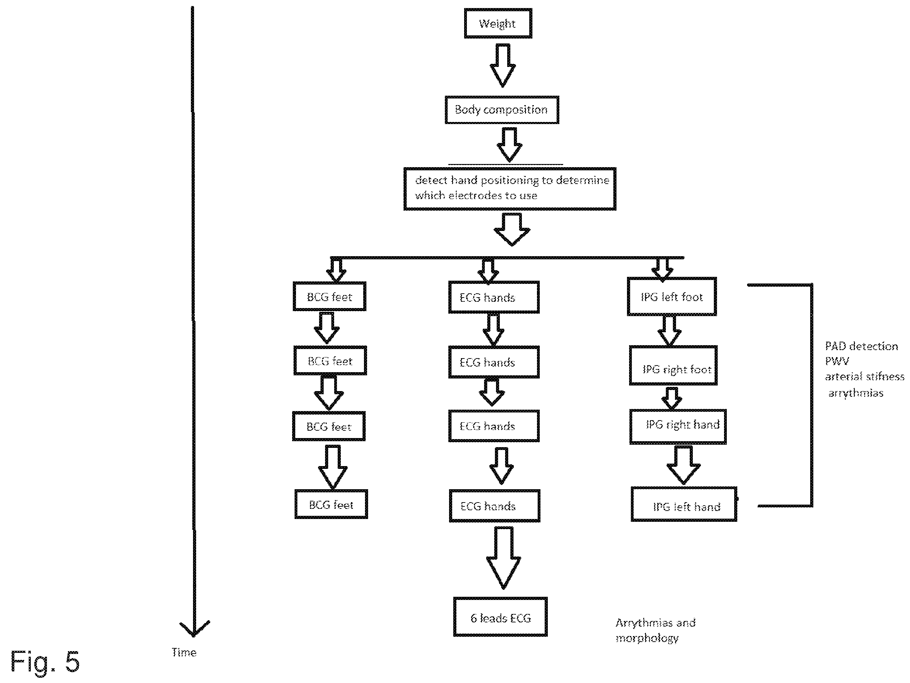

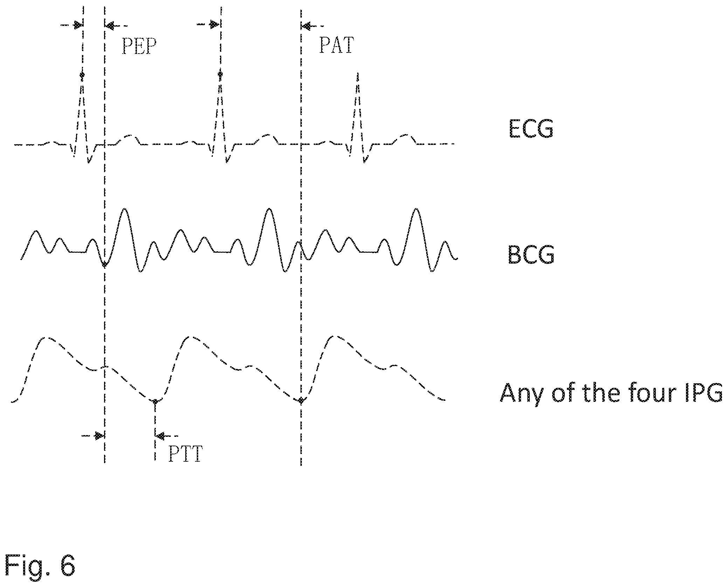

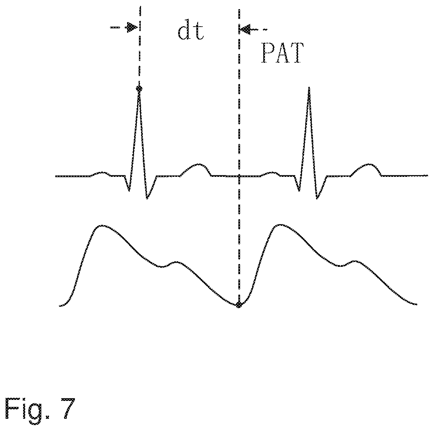

In an embodiment, the weighing scale 100 is configured to acquire the plurality of biometric signals in the sequence illustrated in figure 5. Some exemplary biometric signals are shown in figures 6 and 7. In the sequence shown in figure 5, the weight and body composition of the user 180 are measured first. Then hands and feet positioning may be performed for determining which of the pluralities of electrodes to use. Then BCG and ECG (between the hands) measurements may be performed at all time while the local blood variation measurement of each foot and hand may be done consecutively to lower the electronic burden. Finally, a 6 lead ECG may be performed. The ECG performed at all times may be used to synchronize the various local blood volume variation signals acquired by timing calculation between the R peak of the ECG signal and the points of interest of the PPG/IPG signals (maxima, minima, feet of the wave, ...), as shown for example in figures 6 and 7. The ECG may also enable to separate the other acquired biometric signals into heartbeat, as shown in figure 6, thus simplifying the quality checking step of the acquired signals.

This combination of measurements enables the calculation of the pulse arrival time (PAT) at each hand and each foot, the pulse transit time (PTT) between hand and foot and the pulse transit time between the heart and each hand and each foot, as illustrated in figures 6 and 7 and as will be described with more mathematical details below. Then assuming that the length travelled by the pulse wave during these various timings is proportional to the height of the user 180 with proportional coefficients that can be found during measurement campaigns done during the development of the weighing scale 100, the PWV can be found for the various parts of the body knowing the height and possibly the gender and/or age of the user 180. These values may be used by the processing circuitry 148a of the weighing scale 100 to determine the PWV of the user 100 but also the risk of having PAD by calculation of ratios between arms and legs and check of asymmetries between both arms and both legs (PAT, PTT and PWV can be used for this measurement, calibration may show the most accurate one).

More specifically, in an embodiment the weighing scale 100 and the processing circuitry 148a thereof may determine the biometric signals listed in the following table.

| Measurement 1 | Measurement 2 | Timing obtained |
| --- | --- | --- |
| BCG (feet) | ECG (hands) | Pre ejection time (PEP) |
| PPG/IPG (each hand) | ECG (hands) | Pulse arrival time (PAT) for each hand |
| IPG (each foot) | ECG (hands) | PAT for each foot |
| IPG/PPG (each hand) Synchronized with hands ECG | IPG (each foot) Synchronized with hands ECG | Pulse transit time (PTT) arm to foot |

Based on these signals and measurements obtained by the weighing scale 140 the processing circuitry 148a thereof may obtain the three following pulse transit times:

- 

$$PTT_{(heart - hand)} = PAT_{(hand)} - PEP$$

- 

$$PTT_{(heart - foot)} = PAT_{(foot)} - PEP$$

- 

$$PTT_{(hand - foot)}$$

If we consider that the pulse transit time is equal to the speed of the pulse propagation times the distance it travels between the sites we obtain and separating the body in three different PWV values for the trunk, the arms and the legs:

- 

$$PTT_{(heart - hand)} = L_{(trunk\_1)} \times PWV_{(trunk)} + L_{(arm)} \times PWV_{(arm)}$$

- 

$$PTT_{(heart - foot)} = L_{(trunk\_2)} \times PWV_{(trunk)} + L_{(leg)} \times PWV_{(leg)}$$

- 

$$PTT_{(hand - foot)} = (L_{(trunk\_2)} - L_{(trunk\_1)}) \times PWV_{(trunk)} + L_{(leg)} \times PWV_{(leg)} - L_{(arm)} \times PWV_{(arm)}$$

Now we can consider that the different distances $L_i$ can be determined by calibration as proportional to the size of the user such that:

-

$$L_{(trunk\_1)} = \alpha_1 \text{ x height}$$

$$L_{(trunk\_2)} = \alpha_2 \text{ x height}$$

$$L_{(arm)} = \alpha_3 \text{ x height}$$

$$L_{(leg)} = \alpha_4 \text{ x height}$$

The processing circuitry 148a of the weighing scale 100 may then use the following system of equations:

$$PTT_{(heart-hand)} = \text{height x } [\alpha_1 \text{ x } PWV_{(trunk)} + \alpha_3 \text{ x } PWV_{(arm)}]$$

$$PTT_{(heart-foot)} = \text{height x } [\alpha_2 \text{ x } PWV_{(trunk)} + \alpha_4 \text{ x } PWV_{(leg)}]$$

$$PTT_{(hand-foot)} = \text{height x } [(\alpha_2 - \alpha_1) \text{ x } PWV_{(trunk)} + \alpha_4 \text{ x } PWV_{(leg)} - \alpha_3 \text{ x } PWV_{(arm)}]$$

Thus, the processing circuitry 148a of the weighing scale 100 may use these 3 equations with three unknown variables for determining the true value of $PWV_{(trunk)}$, i.e. the pulse wave velocity in the trunk of the user 180. This improvement of the PWV measurement by means of the weighing scale 100 allows for a more precise PAD risk estimate for the user 180. This is because, first the PAT, PTT or PWV differences observed between the two legs and two arms will indicate a possible blockage of one of the limbs. Secondly, a too large difference of the PWV between the legs and the arms can also indicate such problems, which the weighing scale 100 is capable of detecting.

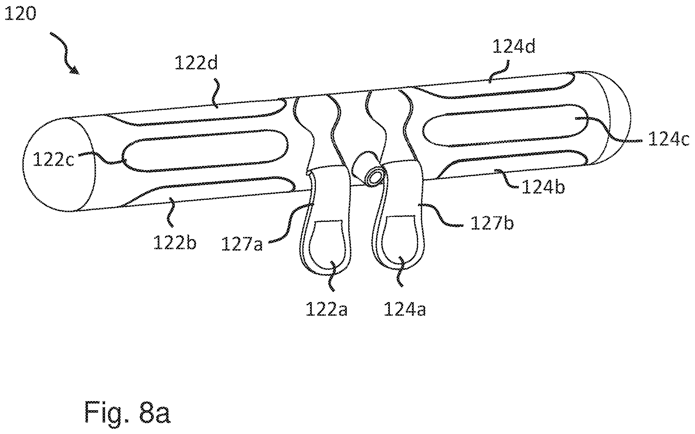

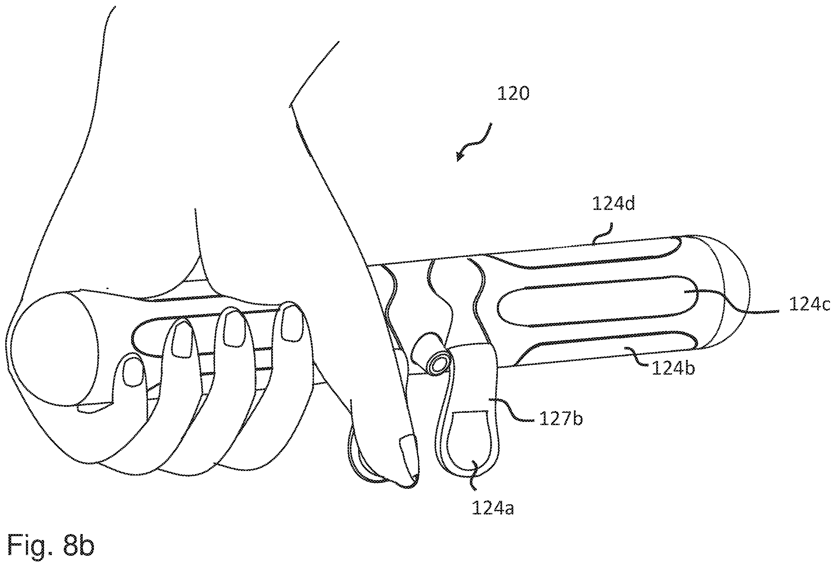

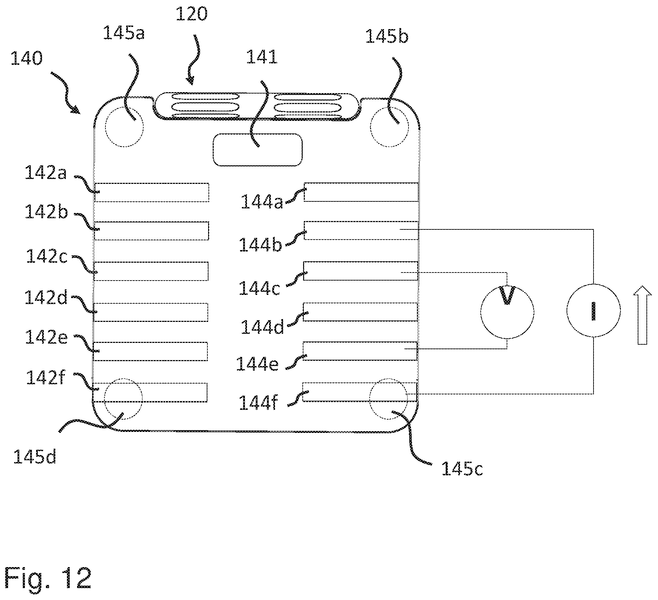

In an embodiment, the base, i.e. weighing platform 140 may comprise a plurality, in particular four load cells 145a-d located at the corners of the base 140 for measuring the weight as well as rapid weight variations of the user 180 for measuring the BCG signals. As shown in figures 2 and 12, the third group of electrodes 142a-f and the fourth group of electrodes 144a-f may comprise each at least 4 electrodes (i.e. at least 4 electrodes per foot) to perform local IPG measurements on each foot. The first and second group of electrodes arranged within the one or more handles 120 may perform ECG signal measurements between the hands and may perform either IPG on each hand (in an embodiment where the first and second group of electrodes comprise at least 5 electrodes, as shown in figures 8a and 8b) or PPG in an embodiment containing 2 electrodes per handle (required for body composition analysis) and at least one PPG sensor 126a,b per hand (as shown in figure 4). In a further embodiment, the processing circuitry 148a of the weighing scale 100 may be further configured to determine the body composition of the user 180 based on the plurality of biometric signals. For such a body composition analysis the first and second group of electrodes arranged within the one or more handles 120 may each comprise at least two electrodes.

In an embodiment, the processing circuitry 148a of the weighing scale 100 may use IPG signal measurements for determining local blood volume variations on the hands of the user 180. To this end, in an embodiment, 5 electrodes are provided for each hand in order to measure ECG and IPG simultaneously on one hand. Because the electrical loop of both measurements should be kept separate, a possible embodiment for the one or more handles 120 is shown in figures 8a and 8b. In this embodiment the one or more handles 120 may have a particular shape with one or more flexible tabs 127a, 127b close to the center of the handles 120 configured for contacting the base of the thumbs therewith. The electrodes placed on the tabs 127a, 127b, i.e. the electrodes 122a and 124a are configured to measure ECG signals, while the other ones (4 for each hand), i.e. 122b-d and 124b-d are placed on the handle bar 120 so that they contact the hands further away along the handle 120 in order to allow measuring the IPG signals.

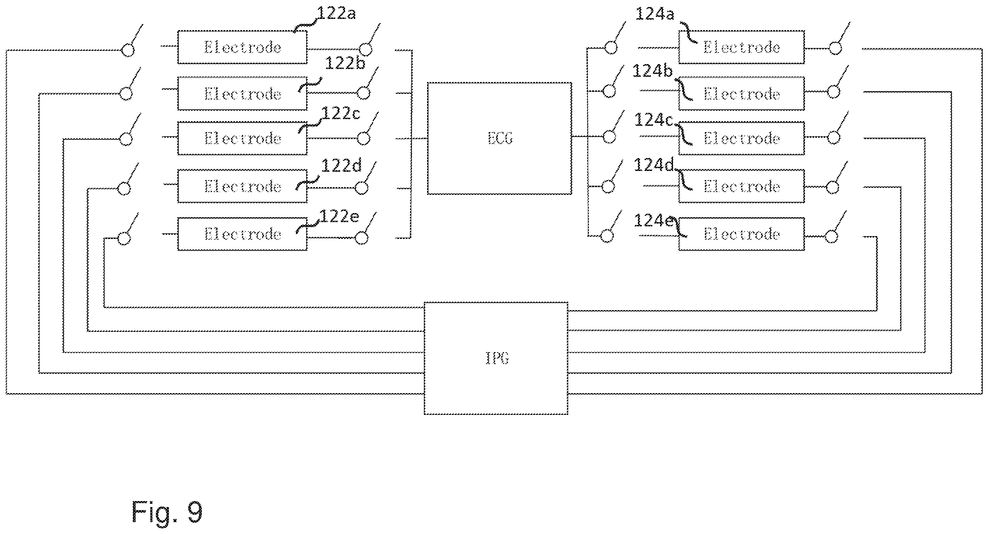

In a further embodiment, IPG is used to determine local blood volume variations on the hands and a classic formfactor may be used for the handle bar(s) 120 as shown in figure 3. In this case also 5 electrodes 122a-g, 124a-g per hand are provided as in the previous embodiment. However, in this embodiment, the weighing scale 100 may further comprise a mechanism for determining the positioning of the hands of the user 180 relative to the electrodes 122a-g, 124a-g. To this end, in an embodiment, the weighing scale 100 is configured to perform an impedance measurement between the electrodes 122a-g of one hand and the electrodes 124a-g of the other hand to determine their positioning, knowing that the further away an electrode is from the wrist the more the impedance will increase because of the increasing electrical path. In order to do so and adapt the role of the electrode depending on its position compared to the hand, an electronic circuitry as illustrated in figure 9 may be implemented by the weighing scale 140. As can be taken from the electronic circuitry shown in figure 9, a respective switch may be placed before each electrode 122a-g, 124a-g in order to contact it to the right measurement unit.

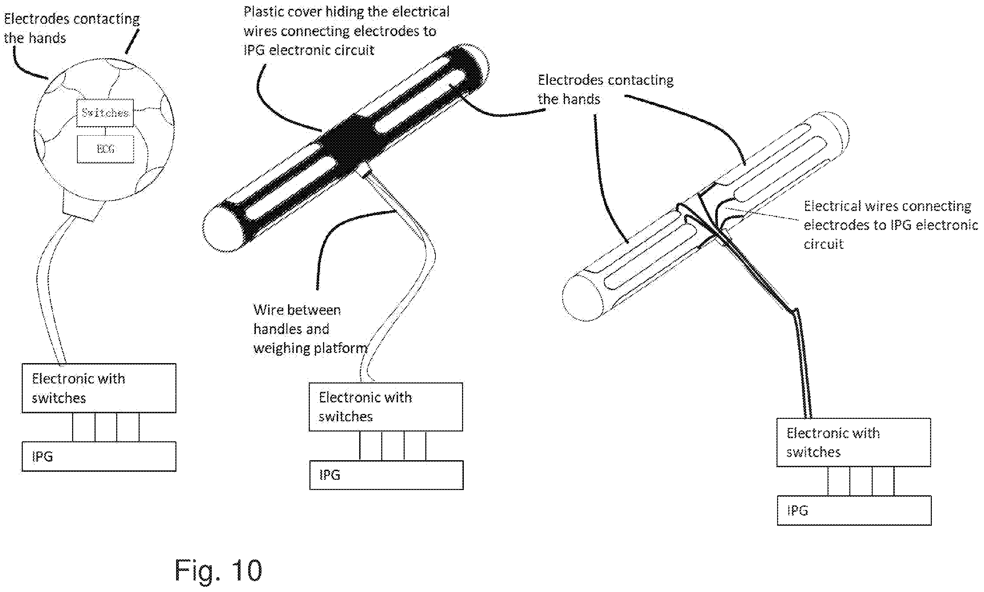

In order to reduce the electrical noise coming from the two simultaneous measurements described in the context of the previous embodiments, in a further embodiment shown in figure 10 the electronic circuitry of the ECG and the IPG measurement are separated. In this embodiment the electronic circuit of the ECG may be placed in the one or more handles 120 contacting the electrodes from the inside thereof, while the IPG electronic circuitry may be placed in the base 140 of the weighing scale 100, contacting the electrodes via the wire 125 connecting the base 140 and the one or more handles 120 as well as the external parts thereof. An isolating cap may be placed on top of the wires coming from the outside of the one or more handles 120 to protect them.

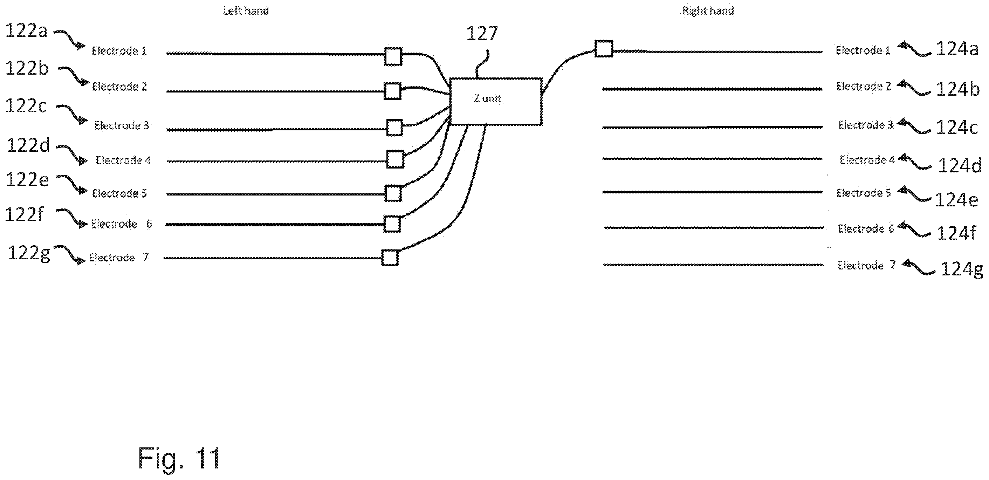

In a further embodiment more than 5 electrodes per hand may be provided on the one or more handles 120 to measure the ECG and the IPG signals. In such an embodiment the positioning of the hands of the user 180 relative to the electrodes 122a-g, 124a-g may be determined in the way described above, i.e. by measurement of the impedance between the electrodes of one hand and the ones of the other. However, a measurement of the impedance between the electrodes of the respective hand may help to determine the best contacting electrodes, as illustrated in figure 11. In this case, the 5 left hand electrodes 122a-g showing the lowest contact impedance (as measured by the impedance unit 127) may be used for the ECG and IPG signal measurements. The electrodes used for the ECG measurement may be the best contacting electrodes closest to the wrist of the user 180, while the IPG may be done using the four (per hand) best contacting electrodes placed further away along the hand.

As shown in figure 12, in an embodiment, the base 140 of the weighing scale 100 may have more than 4 electrodes per foot, for instance six electrodes 142a-f for the left foot and six electrodes 144a-f for the right foot, respectively. In that case an electronic circuitry based on the same principle as the electronic circuitry for the hand electrodes described above may be implemented for contacting the correct electrodes 142a-f, 144a-f depending on the measurement made. An embodiment of this circuitry 148 is shown in figure 13. The IPG signals measurements 148c may be done with the four best contacting electrodes for both feet. In a possible embodiment the signal of the four load cells 145a-d may be analyzed to determine whether the user 180 is more or less on the front or the back part of the base 140 and choose the electrodes most likely to be in contact with the user's feet.

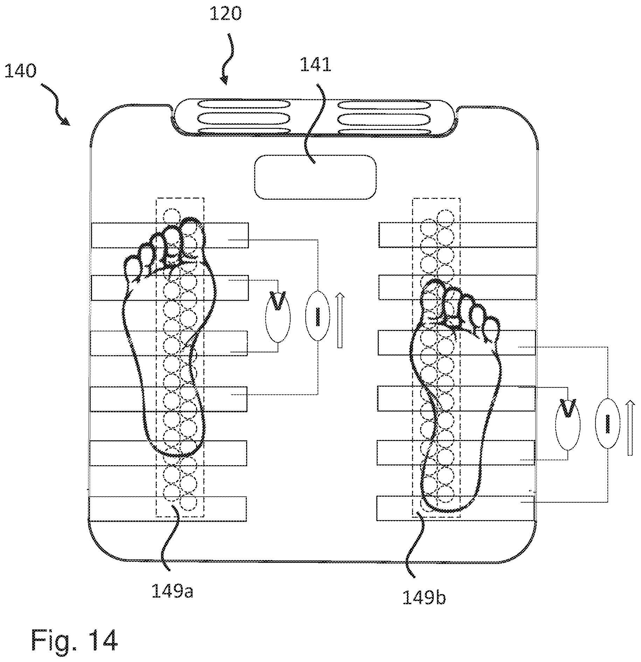

In an embodiment, where the base 140 has more than four electrodes per foot, light sensing may be used to determine the feet positioning relative to the electrodes, as illustrated in figure 14. In this embodiment, the top surface, i.e. cover of the base 140 may be made from glass and the electrodes 142a-f, 144a-f may comprise ITO (Indium Tin Oxyde) or any other transparent electrode material. LEDs and photodiodes either as single elements under the electrodes or as arrays underneath the base cover may be used to detect the position, i.e. the "shadow" of the feet and determine on which electrodes the feet are placed, as illustrated in figure 14. The electrodes 142a-f, 144a-f receiving the least amount of ambient light (because of the shadow cast by the users' feet) or the most amount of reflected light (in case a combination of LEDs and photodiodes are used) are the ones presenting the lowest contact impedance for the feet and, therefore, may be chosen to improve the IPG signal quality.

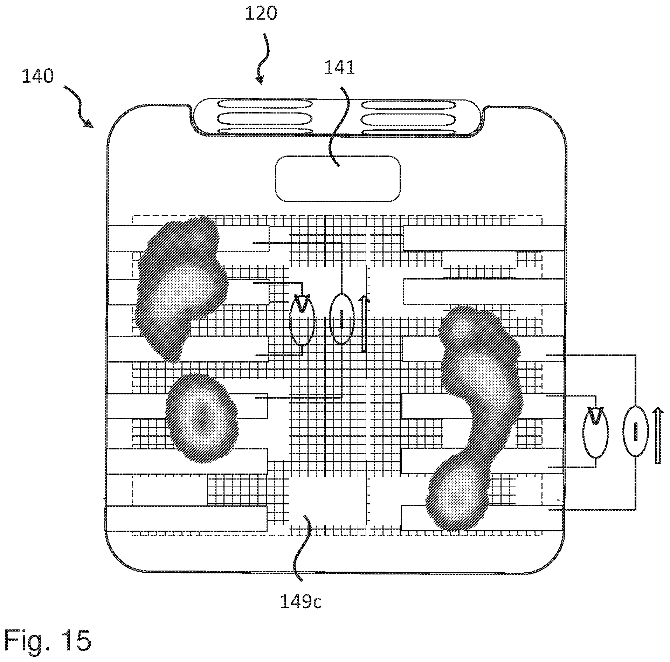

In a further embodiment shown in figure 15, the base 140 may comprise a pressure sensor array or a pressure mat 149c for determining the best contacting electrodes. The electrodes that will be selected for the IPG measurement will be the ones showing the most pressure indicating good contact between the feet and the electrodes.

In an embodiment, the electrodes 142a-f, 144a-f for the feet of the user 180 may be used to perform a 6 lead ECG using the electrodes of the feet and the hands. In an embodiment, the processing circuitry 148 of the weighing scale 140 is configured to connect the electrodes of one limb together in order to decrease the contact impedance in the case of body composition analysis or six lead ECG.

Thus, embodiments of the weighing scale 100 disclosed herein allow detecting PAD by means of an everyday electronic consumer product. Moreover, embodiments of the weighing scale 100 disclosed herein provide a more precise PWV estimate due to the PWV determination by limb and not globally for the whole body of the user and a complete cardiovascular health assessment by combining PAD detection, PWV measurement and 6 lead ECG measurement.

The person skilled in the art will understand that the "blocks" ("units") of the various figures (method and apparatus) represent or describe functionalities of embodiments of the present disclosure (rather than necessarily individual "units" in hardware or software) and thus describe equally functions or features of apparatus embodiments as well as method embodiments (unit = step).

In the several embodiments provided in the present application, it should be understood that the disclosed system, apparatus, and method may be implemented in other manners. For example, the described embodiment of an apparatus is merely exemplary. For example, the unit division is merely logical function division and may be another division in an actual implementation. For example, a plurality of units or components may be combined or integrated into another system, or some features may be ignored or not performed. In addition, the displayed or discussed mutual couplings or direct couplings or communication connections may be implemented by using some interfaces. The indirect couplings or communication connections between the apparatuses or units may be implemented in electronic, mechanical, or other forms.

The units described as separate parts may or may not be physically separate, and parts displayed as units may or may not be physical units, may be located in one position, or may be distributed on a plurality of network units. Some or all of the units may be selected according to actual needs to achieve the objectives of the solutions of the embodiments.

In addition, functional units in the embodiments of the invention may be integrated into one processing unit, or each of the units may exist alone physically, or two or more units are integrated into one unit.

**Claims**

1. A weighing scale (100) for monitoring biometric data of a user (180), wherein the weighing scale (100) comprises:

   a first group (122a-g) of a plurality of electrodes configured to be brought into contact with a first hand of the user (180);
   a second group (124a-g) of the plurality of electrodes configured to be brought into contact with a second hand of the user (180);
   one or more handles (120) configured to be gripped by the first and second hand of the user (180), wherein the first group (122a-g) of the plurality of electrodes and the second group (124a-g) of the plurality of electrodes are arranged on the one or more handles (120);
   a base (140) configured to support the user (180) and determine a weight of the user (180), wherein a first portion of a top surface of the base (140) comprises a third group (142a-f) of the plurality of electrodes configured to be brought into contact with a first foot of the user (180) and a second portion of the top surface of the base (140) comprises a fourth group (144a-f) of the plurality of electrodes configured to be brought into contact with a second foot of the user (180); and
   a processing circuitry (148a) configured to obtain a plurality of biometric signals of the user (180), including one or more electrocardiogram, ECG, signals for one or more ECG leads defined by the plurality of electrodes, a plurality of photoplethysmography, PPG, and impedance plethysmography, IPG, signals for the hands and the feet of the user (180) for determining local blood volume variations;
   wherein the processing circuitry (148a) is further configured to determine, based on the plurality of biometric signals, a pulse arrival time, PAT, and to determine, based on the PAT, a pulse wave velocity, PWV, for the first and second hand and the first and second foot of the user (180) for estimating a peripheral arterial disease, PAD, risk of the user (180); and
   **characterized in that**
   the first group (122a-g) of the plurality of electrodes comprises at least 5 electrodes (122a-g) and/or wherein the second group (124a-g) of the plurality of electrodes comprises at least 5 electrodes (124a-g) for simultaneous measurement of local IPG on each hand and ECG between the hands of the user (180).

2. The weighing scale (100) of claim 1, wherein the plurality of biometric signals of the user (180) further include one or more ballistocardiography, BCG, signals of the user (180) and wherein the processing circuitry (148a) is configured to obtain the one or more BCG signals on the basis of weight variations of the user (180) determined by the base (140).

3. The weighing scale (100) of claim 2, wherein the processing circuitry (148a) is further configured to determine a pre-ejection period on the basis of the one or more ECG signals and the one or more BCG signals.

4. The weighing scale (100) of claim 2 or 3, wherein the processing circuitry (148a) is further configured to determine a pulse transit time, PTT, and the PWV for the first and second hand and the first and second foot of the user (180) for estimating the peripheral arterial disease, PAD, risk of the user (180).

5. The weighing scale (100) of any one of the preceding claims, wherein the base (140) further comprises at least one light sensor (149a,b) configured to detect the position of the feet of the user (180) relative to the third group (142a-f) and the fourth group (144a-f) of the plurality of electrodes.

6. The weighing scale (100) of any one of the preceding claims, wherein the base (140) comprises one or more load cells (145a-d) configured to determine the weight of the user (180).

7. The weighing scale (100) of any one of the preceding claims, wherein the weighing scale (100) further comprises a user interface (141) and/or a communication interface configured to output information associated with one or more of the plurality of biometric signals to the user (180) and/or configured to receive information about a height, age and/or sex of the user (180).

8. The weighing scale (100) of any one of the preceding claims, wherein the third group (142a-f) of the plurality of electrodes comprises at least 4 electrodes (142a-f) and wherein the fourth group (144a-f) of the plurality of electrodes comprises at least 4 electrodes (144a-f) for a local IPG measurement on each foot.

9. The weighing scale (100) of any one of the preceding claims, wherein the one or more handles (120) comprises at least one PPG sensor (126a,b) for each handle (120) and wherein the first group (122a-g) of the plurality of electrodes comprises at least 1 electrode (122a-g) and/or wherein the second group (124a-g) of the plurality of electrodes comprises at least 1 electrode (124a-g) for simultaneous measurement of ECG between the hands and PPG on each hand of the user (180).

10. The weighing scale (100) of claim 9, wherein the processing circuitry (148a) is further configured to determine a first position of the first hand of the user (180) and a second position of the second hand of the user (180) on the one or more handles (120) and to select, based on the first position and the second position, one or more of the at least 5 electrodes of the first group (122a-g) of the plurality of electrodes and/or one or more of the at least 5 electrodes of the second group (124a-g) of the plurality of electrodes for obtaining the plurality of biometric signals of the user (180).

11. The weighing scale (100) of any one of the preceding claims, wherein the base (140) further comprises a pressure sensor array (149) configured to detect the position of the first feet and the second feet of the user (180) on the base (140).

12. The weighing scale (100) of any one of the preceding claims, wherein the processing circuitry (148a) is further configured to determine, based on the plurality of biometric signals, the pulse wave velocity, PWV, in the trunk of the user (180).

13. The weighing scale (100) of any one of the preceding claims, wherein the processing circuitry (148a) is configured to obtain the plurality of ECG signals for at least 6 ECG leads defined by the plurality of electrodes and to determine, based on the plurality of ECG signals, a heart morphology of the user (180) and/or enable an opportunistic detection of arrythmias.

**Patentansprüche**

1. Waage (100) zur Überwachung biometrischer Daten eines Benutzers (180), wobei die Waage (100) Folgendes umfasst:

   eine erste Gruppe (122a-g) einer Vielzahl von Elektroden, die dazu konfiguriert ist, mit einer ersten Hand des Benutzers (180) in Kontakt gebracht zu werden;
   eine zweite Gruppe (124a-g) der Vielzahl von Elektroden, die dazu konfiguriert ist, mit einer zweiten Hand des Benutzers (180) in Kontakt gebracht zu werden;
   einen oder mehrere Griffe (120), die dazu konfiguriert sind, durch die erste und die zweite Hand des Benutzers (180) ergriffen zu werden, wobei die erste Gruppe (122a-g) der Vielzahl von Elektroden und die zweite Gruppe (124a-g) der Vielzahl von Elektroden an dem einen oder den mehreren Griffen (120) angeordnet sind;
   eine Basis (140), die dazu konfiguriert ist, den Benutzer (180) zu stützen und ein Gewicht des Benutzers (180) zu bestimmen, wobei ein erster Abschnitt einer Oberseite der Basis (140) eine dritte Gruppe (142a-f) der Vielzahl von Elektroden umfasst, die dazu konfiguriert ist, mit einem ersten Fuß des Benutzers (180) in Kontakt gebracht zu werden, und ein zweiter Abschnitt der Oberseite der Basis (140) eine vierte Gruppe (144a-f) der Vielzahl von Elektroden umfasst, die dazu konfiguriert ist, mit einem zweiten Fuß des Benutzers (180) in Kontakt gebracht zu werden; und
   eine Verarbeitungsschaltung (148a), die dazu konfiguriert ist, eine Vielzahl von biometrischen Signalen des Benutzers (180), beinhaltend ein oder mehrere Elektrokardiogrammsignale, EKG-Signale, für eine oder mehrere EKG-Ableitungen, die durch die Vielzahl von Elektroden definiert sind, eine Vielzahl von Photoplethysmographie-Signalen, PPG-Signalen, und Impedanzplethysmographie-Signalen, IPG-Signalen, für die Hände und die Füße des Benutzers (180) zu erlangen, um lokale Blutvolumenschwankungen zu bestimmen;

wobei die Verarbeitungsschaltung (148a) ferner dazu konfiguriert ist, basierend auf der Vielzahl von biometrischen Signalen eine Pulsankunftszeit, PAT, zu bestimmen und basierend auf der PAT eine Pulswellengeschwindigkeit, PWV, für die erste und die zweite Hand und den ersten und den zweiten Fuß des Benutzers (180) zu bestimmen, um ein Risiko des Benutzers (180) für eine periphere arterielle Verschlusskrankheit, PAVK, abzuschätzen; und

**dadurch gekennzeichnet, dass** die erste Gruppe (122a-g) der Vielzahl von Elektroden mindestens 5 Elektroden (122a-g) umfasst und/oder wobei die zweite Gruppe (124a-g) der Vielzahl von Elektroden mindestens 5 Elektroden (124a-g) zur gleichzeitigen lokalen IPG-Messung an jeder Hand und EKG-Messung zwischen den Händen des Benutzers (180) umfasst.

2. Waage (100) nach Anspruch 1, wobei die Vielzahl von biometrischen Signalen des Benutzers (180) ferner ein oder mehrere Ballistokardiographie-Signale, BCG-Signale, des Benutzers (180) beinhaltet und wobei die Verarbeitungsschaltung (148a) dazu konfiguriert ist, das eine oder die mehreren BCG-Signale basierend auf Gewichtsschwankungen des Benutzers (180), die durch die Basis (140) bestimmt wurden, zu erlangen.

3. Waage (100) nach Anspruch 2, wobei die Verarbeitungsschaltung (148a) ferner dazu konfiguriert ist, eine Zeitspanne vor der Auswurfphase basierend auf dem einen oder den mehreren EKG-Signalen und dem einen oder den mehreren BKG-Signalen zu bestimmen.

4. Waage (100) nach Anspruch 2 oder 3, wobei die Verarbeitungsschaltung (148a) ferner dazu konfiguriert ist, eine Pulswellenlaufzeit (PTT) und die PWV für die erste und die zweite Hand und den ersten und den zweiten Fuß des Benutzers (180) zu bestimmen, um das Risiko des Benutzers (180) für eine periphere arterielle Verschlusskrankheit, PAVK, abzuschätzen.

5. Waage (100) nach einem der vorhergehenden Ansprüche, wobei die Basis (140) ferner mindestens einen Lichtsensor (149a,b) umfasst, der dazu konfiguriert ist, die Position der Füße des Benutzers (180) relativ zu der dritten Gruppe (142a-f) und der vierten Gruppe (144a-f) der Vielzahl von Elektroden zu erkennen.

6. Waage (100) nach einem der vorhergehenden Ansprüche, wobei die Basis (140) eine oder mehrere Wägezellen (145a-d) umfasst, die dazu konfiguriert sind, das Gewicht des Benutzers (180) zu bestimmen.

7. Waage (100) nach einem der vorhergehenden Ansprüche, wobei die Waage (100) ferner eine Benutzerschnittstelle (141) und/oder eine Kommunikationsschnittstelle umfasst, die dazu konfiguriert ist/sind, Informationen, die mit einem oder mehreren der Vielzahl von biometrischen Signalen verknüpft sind, an den Benutzer (180) auszugeben und/oder dazu konfiguriert ist/sind, Informationen über Größe, Alter und/oder Geschlecht des Benutzers (180) zu empfangen.

8. Waage (100) nach einem der vorhergehenden Ansprüche, wobei die dritte Gruppe (142a-f) der Vielzahl von Elektroden mindestens 4 Elektroden (142a-f) umfasst und wobei die vierte Gruppe (144a-f) der Vielzahl von Elektroden mindestens 4 Elektroden (144a-f) für eine lokale IPG-Messung an jedem Fuß umfasst.

9. Waage (100) nach einem der vorhergehenden Ansprüche, wobei der eine oder die mehreren Griffe (120) mindestens einen PPG-Sensor (126a,b) für jeden Griff (120) umfasst/umfassen und wobei die erste Gruppe (122a-g) der Vielzahl von Elektroden mindestens 1 Elektrode (122a-g) umfasst und/oder wobei die zweite Gruppe (124a-g) der Vielzahl von Elektroden mindestens 1 Elektrode (124a-g) zur gleichzeitigen EKG-Messung zwischen den Händen und PPG-Messung an jeder Hand des Benutzers (180) umfasst.

10. Waage (100) nach Anspruch 9, wobei die Verarbeitungsschaltung (148a) ferner dazu konfiguriert ist, eine erste Position der ersten Hand des Benutzers (180) und eine zweite Position der zweiten Hand des Benutzers (180) auf dem einen oder den mehreren Griffen (120) zu bestimmen und basierend auf der ersten Position und der zweiten Position eine oder mehrere der mindestens 5 Elektroden der ersten Gruppe (122a-g) der Vielzahl von Elektroden und/oder eine oder mehrere der mindestens 5 Elektroden der zweiten Gruppe (124a-g) der Vielzahl von Elektroden auszuwählen, um die Vielzahl von biometrischen Signalen des Benutzers (180) zu erlangen.

11. Waage (100) nach einem der vorhergehenden Ansprüche, wobei die Basis (140) ferner eine Drucksensoranordnung (149) umfasst, die dazu konfiguriert ist, die Position des ersten Fußes und des zweiten Fußes des Benutzers (180) auf der Basis (140) zu erkennen.

**12.** Waage (100) nach einem der vorhergehenden Ansprüche, wobei die Verarbeitungsschaltung (148a) ferner dazu konfiguriert ist, basierend auf der Vielzahl von biometrischen Signalen die Pulswellengeschwindigkeit, PWV, in dem Rumpf des Benutzers (180) zu bestimmen.

**13.** Waage (100) nach einem der vorhergehenden Ansprüche, wobei die Verarbeitungsschaltung (148a) dazu konfiguriert ist, die Vielzahl von EKG-Signalen für mindestens 6 EKG-Ableitungen, die durch die Vielzahl von Elektroden definiert sind, zu erlangen und basierend auf der Vielzahl von EKG-Signalen eine Herzmorphologie des Benutzers (180) zu bestimmen und/oder eine opportunistische Erkennung von Arrhythmien zu ermöglichen.

**Revendications**

**1.** Balance (100) permettant de surveiller les données biométriques d'un utilisateur (180), dans laquelle la balance (100) comprend :

un premier groupe (122a-g) d'une pluralité d'électrodes configurées pour être mises en contact avec une première main de l'utilisateur (180) ;
un deuxième groupe (124a-g) de la pluralité d'électrodes configurées pour être mises en contact avec une seconde main de l'utilisateur (180) ;
une ou plusieurs poignées (120) configurées pour être saisies par la première et la seconde main de l'utilisateur (180), dans laquelle le premier groupe (122a-g) de la pluralité d'électrodes et le deuxième groupe (124a-g) de la pluralité d'électrodes sont agencés sur les une ou plusieurs poignées (120) ;
une base (140) configurée pour supporter l'utilisateur (180) et déterminer un poids de l'utilisateur (180), dans laquelle une première partie de la surface supérieure de la base (140) comprend un troisième groupe (142a-f) de la pluralité d'électrodes configurées pour être mises en contact avec un premier pied de l'utilisateur (180) et une seconde partie de la surface supérieure de la base (140) comprend un quatrième groupe (144a-f) de la pluralité d'électrodes configurées pour être mises en contact avec un second pied de l'utilisateur (180) ; et
un circuit de traitement (148a) configuré pour obtenir une pluralité de signaux biométriques de l'utilisateur (180), comportant un ou plusieurs signaux d'électrocardiogramme, ECG, pour une ou plusieurs dérivations ECG définies par la pluralité d'électrodes, une pluralité de signaux de photopléthysmographie, PPG, et de pléthysmographie d'impédance, IPG, pour les mains et les pieds de l'utilisateur (180) pour déterminer les variations locales du volume sanguin ;
dans laquelle le circuit de traitement (148a) est également configuré pour déterminer, sur la base de la pluralité de signaux biométriques, un temps d'arrivée d'impulsion, PAT, et pour déterminer, sur la base du PAT, une vitesse d'onde de pouls, PWV, pour la première et la seconde main et le premier et le second pied de l'utilisateur (180) pour estimer un risque de maladie artérielle périphérique, MAP, de l'utilisateur (180) ; et
**caractérisée en ce que** le premier groupe (122a-g) de la pluralité d'électrodes comprend au moins 5 électrodes (122a-g) et/ou dans laquelle le deuxième groupe (124a-g) de la pluralité d'électrodes comprend au moins 5 électrodes (124a-g) pour la mesure simultanée de l'IPG local sur chaque main et de l'ECG entre les mains de l'utilisateur (180).

**2.** Balance (100) selon la revendication 1, dans laquelle la pluralité de signaux biométriques de l'utilisateur (180) comportent également un ou plusieurs signaux de balistocardiographie, BCG, de l'utilisateur (180) et dans laquelle le circuit de traitement (148a) est configuré pour obtenir les un ou plusieurs signaux BCG sur la base des variations de poids de l'utilisateur (180) déterminées par la base (140).

**3.** Balance (100) selon la revendication 2, dans laquelle le circuit de traitement (148a) est également configuré pour déterminer une période de pré-éjection sur la base des un ou plusieurs signaux ECG et des un ou plusieurs signaux BCG.

**4.** Balance (100) selon la revendication 2 ou 3, dans laquelle le circuit de traitement (148a) est également configuré pour déterminer un temps de transit d'impulsion, PTT, et le PWV pour la première et la seconde main et le premier et le second pied de l'utilisateur (180) pour estimer le risque de maladie artérielle périphérique, MAP, de l'utilisateur (180).

**5.** Balance (100) selon l'une quelconque des revendications précédentes, dans laquelle la base (140) comprend également au moins un capteur de lumière (149a,b) configuré pour détecter la position des pieds de l'utilisateur (180) par rapport au troisième groupe (142a-f) et au quatrième groupe (144a-f) de la pluralité d'électrodes.

6. Balance (100) selon l'une quelconque des revendications précédentes, dans laquelle la base (140) comprend une ou plusieurs cellules de charge (145a-d) configurées pour déterminer le poids de l'utilisateur (180).

7. Balance (100) selon l'une quelconque des revendications précédentes, dans laquelle la balance (100) comprend également une interface utilisateur (141) et/ou une interface de communication configurée pour délivrer en sortie des informations associées à l'un ou plusieurs de la pluralité de signaux biométriques à l'utilisateur (180) et/ou configurée pour recevoir des informations sur la taille, l'âge et/ou le sexe de l'utilisateur (180).

8. Balance (100) selon l'une quelconque des revendications précédentes, dans laquelle le troisième groupe (142a-f) de la pluralité d'électrodes comprend au moins 4 électrodes (142a-f) et dans laquelle le quatrième groupe (144a-f) de la pluralité d'électrodes comprend au moins 4 électrodes (144a-f) pour une mesure IPG locale sur chaque pied.

9. Balance (100) selon l'une quelconque des revendications précédentes, dans laquelle les une ou plusieurs poignées (120) comprennent au moins un capteur PPG (126a,b) pour chaque poignée (120) et dans laquelle le premier groupe (122a-g) de la pluralité d'électrodes comprend au moins une électrode (122a-g) et/ou dans laquelle le deuxième groupe (124a-g) de la pluralité d'électrodes comprend au moins une électrode (124a-g) pour la mesure simultanée de l'ECG entre les mains et du PPG sur chaque main de l'utilisateur (180).

10. Balance (100) selon la revendication 9, dans laquelle le circuit de traitement (148a) est également configuré pour déterminer une première position de la première main de l'utilisateur (180) et une seconde position de la seconde main de l'utilisateur (180) sur les une ou plusieurs poignées (120) et pour sélectionner, sur la base de la première position et de la seconde position, l'une ou plusieurs des au moins 5 électrodes du premier groupe (122a-g) de la pluralité d'électrodes et/ou l'une ou plusieurs des au moins 5 électrodes du deuxième groupe (124a-g) de la pluralité d'électrodes pour obtenir la pluralité de signaux biométriques de l'utilisateur (180).

11. Balance (100) selon l'une quelconque des revendications précédentes, dans laquelle la base (140) comprend également un réseau de capteurs de pression (149) configuré pour détecter la position des premiers pieds et des seconds pieds de l'utilisateur (180) sur la base (140).

12. Balance (100) selon l'une quelconque des revendications précédentes, dans laquelle le circuit de traitement (148a) est également configuré pour déterminer, sur la base de la pluralité de signaux biométriques, la vitesse d'onde de pouls, PWV, dans le tronc de l'utilisateur (180).

13. Balance (100) selon l'une quelconque des revendications précédentes, dans laquelle le circuit de traitement (148a) est configuré pour obtenir la pluralité de signaux ECG pour au moins 6 dérivations ECG définies par la pluralité d'électrodes et pour déterminer, sur la base de la pluralité de signaux ECG, une morphologie cardiaque de l'utilisateur (180) et/ou permettre une détection opportuniste d'arythmies.

100

180

Local IPG/PPG right hand

Local IPG/PPG left hand

120

125

Local IPG right foot

140

Local IPG left foot

Fig. 1

Fig. 2

EP 4 291 084 B1

Fig. 3

Fig. 4

Weight

Body composition

detect hand positioning to determine
which electrodes to use

| BCG feet | ECG hands | IPG left foot |
| BCG feet | ECG hands | IPG right foot |
| BCG feet | ECG hands | IPG right hand |
| BCG feet | ECG hands | IPG left hand |

6 leads ECG

PAD detection
PWV
arterial stifness
arrythmias

Arrythmias and
morphology

Fig. 5

Time

Fig. 6

Fig. 7

Fig. 8a

Fig. 8b

Fig. 9

Fig. 10

Fig. 11

EP 4 291 084 B1

Fig. 12

Fig. 13

Fig. 14

Fig. 15

**EP 4 291 084 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016095522 A1
- US 2016038037 A1
- US 2013289889 A1
- WO 2019134032 A1